# EUROPEAN PATENT SPECIFICATION

(11) **EP 2 009 031 B1**
(45) Date of publication and mention of the grant of the patent: **30.01.2013**
(21) Application number: 08159104.2
(22) Date of filing: 26.06.2008
(51) Int. Cl.: C08F 8/44, C08F 8/42, C08F 26/06, A61K 31/787, A01N 43/40, C08L 39/08, C09D 139/08, D06M 15/356

(54) **Antimicrobial compositions and methods of making same**
Antimikrobielle Zusammensetzungen und Verfahren zu ihrer Herstellung
Compositions antimicrobes et procédés de fabrication de ceux-ci

(30) Priority: 26.06.2007 US 946347 P; 28.06.2007 US 946900 P
(43) Date of publication of application: 31.12.2008
(73) Proprietor: Chuang, Vincent T., Lin-Kou 244 Taiwan (CN)
(72) Inventor: Chuang, Vincent T., Lin-Kou 244 Taiwan (CN)
(74) Representative: Popp, Eugen

(56) References cited:
- WO-A-2007/147094
- KAWABATA N ET AL: "Continuous production of L-aspartic acid from ammonium fumarate using immobilized cells by capture on the surface of nonwoven cloth coated with a pyridinium-type polymer" JOURNAL OF FERMENTATION AND BIOENGINEERING 1995 DEP. OF CHEM. & MATERIALS TECH., FAC. OF ENG. & DESIGN, KYOTO INST. OF TECH., MATSUGASAKI, SAKYO-KU, KYOTO 606, JAPAN, vol. 79, no. 4, 1995, page 317, XP002493786
- OGATA ET AL: "Synthesis, properties, and functions of thermosensitive copolymers having pyridyl and/or pyridinium groups" REACTIVE & FUNCTIONAL POLYMERS, ELSEVIER SCIENCE PUBLISHERS BV, NL, vol. 67, no. 8, 24 May 2007 (2007-05-24), pages 700-707, XP022163218 ISSN: 1381-5148
- CHAUHAN G S ET AL: "Synthesis and characterization of 4-vinyl pyridine-grafted teflon-PFA film for water technologies" JOURNAL OF POLYMER SCIENCE, PART A: POLYMER CHEMISTRY 20001215 JOHN WILEY & SONS INC, vol. 38, no. 24, 15 December 2000 (2000-12-15), pages 4506-4518, XP002493787
- CEN L ET AL: "Antibacterial activity of cloth functionalized with N-alkylated poly(4-vinylpyridine)" JOURNAL OF BIOMEDICAL MATERIALS RESEARCH, vol. 71A, no. 1, 1 October 2004 (2004-10-01), pages 70-80, XP002493788 ISSN: 0021-9304
- VARUN SAMBHY, BLAKE R. PETERSON AND AYUSMAN SEN: "Multifunctional Silane Polymers for Persistent Surface Derivatization and Their Antimicrobial Properties" LANGMUIR, vol. 24, no. 14, 12 June 2008 (2008-06-12), pages 7549-7558, XP002509320 Washington DC, USA
- R. KÜGLER, O. BOULOUSSA, F. RONDELEZ: "Evidence of a charge-density threshold for optimum efficiency of biocidal cationic surfaces" MICROBIOLOGY, vol. 151, May 2005 (2005-05), pages 1341-1348, XP002509321 Reading, United Kingdom

## Description

### FIELD OF THE INVENTION

This invention relates to a process of making a group of silylated poly(N-alkyl-4-vinylpyridinium) quaternized salts suitable for use as coating materials for the treatment of cotton fiber surfaces to impart an antimicrobial effect.

### BACKGROUND OF THE INVENTION

Quaternized polymers of 4-vinylpyridine have been shown to exhibit antibacterial properties both in aqueous solutions and when coated on surfaces to kill airborne microbes. See, e.g., Proc. Natl. Acad. Sci. USA, 98, 5981 (2001); C&EN, May 28, 2001, page 13; Biotechnol. Lett., 24, 801 (2002); Biotechnol. Bioeng., 79, 465 (2002); and Polymeric Materials: Science & Engineering, 91, 814 (2004).

An example of a method for functionalizing a surface so as to impart antibacterial properties is disclosed in U.S. Patent Publ. US-2003-0091641-A1. The disclosed four-step process involves 1) deposition of a SiO₂ nanolayer on a substrate, 2) aminating the surface with 3-aminopropyltrimethoxysilane, 3) bromoalkylating the surface with 1,4-dibromobutane, then 4) derivatizing the bromoalkylated surface with alkyl-polyvinylpyridine in the presence of bromoalkane. A drawback of the disclosed process using 1,4-dibromobutane is that the formation of bis-products inevitably results, resulting in crosslinked surface groups incapable of further functionalization.

KAWABATA N ET AL.: "Continuous production of L-aspartic acid from ammonium fumarate using immobilized cells by capture on the surface of non-woven cloth coated with a pyridinium-type polymer." JOURNAL OF FERMENTATION AND BIOENGINEERING 1995 DEP. OF CHEM. & MATERIALS TECH., FAC. OF ENG. & DESIGN, KYOTO INST. OF TECH., MATSUGASAKI, SAKYO-KU, KYOTO 606, JAPAN, vol. 79, no. 4, 1995, page 317, XP002493786 is directed to the use of immobilized cells on a non-woven cloth in a continuous operation fixed-bed reactor. It is described in this document that poly(N-benzyl-4-vinylpyrridinium chloride-co-styrene) is used for the coating of a non-woven, which is in turn used for capturing cells of Escherichia coli.

OGATA ET AL.: "Synthesis, properties, and functions of thermosensitive copolymers having pyridyl and/or pyridinium groups" REACTIVE & FUNCTIONAL POLYMERS, ELSEVIER SCIENCE PUBLISHERS BV, NL, vol. 67, no. 8, 24 May 2007 (2007-05-24), pagefs 700-707, XP022163218 ISSN: 1381-5148 is concerned with thermosensitive copolymers with pyridyl and/or pyridinium groups.

CHAUHAN G S ET AL.: "Synthesis and characterization of 4-vinyl pyridine-grafted teflon-PFA film water technologies" JOURNAL OF POLYMER SCIENCE, PART A: POLYMER CHEMISTRY 20001215 JOHN WILEY & SONS INC., vol. 38, no. 24, 15 December 2000 (2000-12-15), pages 4506-4518, XP002493787 discloses polymeric materials resulting from the grafting of 4-vinylpyridine onto Teflon-PF.

CEN L ET AL.: "Antibacterial activity of cloth functionalized with N-alkylated poly/4-vinylpyridine)" JOURNAL OF BIOMEDICAL MATERIALS RESEARCH, vol. 71A, no. 1, 1 October 2004 (2004-10-01), pages 70-80m XP002493788 ISSN: 0021-9304 is directed to a cotton cloth functionalized with poly(4-vinyl pyridine).

R. KÜGLER, O. BOLULOUSSA, F. RONDELEZ: "Evidence of a charge-density threshold for optimum efficiency of biocidal cationic surfaces" MICROBIOLOGY, vol. 151, May 2005 (2005-05), pages 1341-1348, XP002509321 Reading, United Kingdom describes the deposition of organic monolayers containing quaternary ammonium groups on solid surfaces. Specifically, the chemical functionalization by poly(4-phenyl-N-butyl pyrridinium) bromide of the surface of fused silica beads, planar glass slides, and oxidized silicon wafers is described. In the end product of this grafting process, a solid material containing SiO₂ is grafted with poly(4-vinyl-N-butyl pyrridinium) polymer chains.

### SUMMARY OF THE INVENTION

Products having antimicrobial surfaces are highly desirable. Such surfaces preferably exhibit an antibacterial effect against both Gram positive bacteria (e.g., *Staphylococcus aureus)* and Gram negative bacteria *(E. coli).* Surfaces that can be treated to impart antimicrobial properties include glass surfaces as well textile surfaces (e.g., cotton). Such surfaces exhibit durability with respect to the antimicrobial layer, specifically, the ability to withstand repeated cleaning/washing cycles.

Likewise, a method of preparing such surfaces that avoids the drawbacks of prior art methods, e.g., cross-linking of surface groups prior to functionalization, is also highly desirable. Such methods employ specific pyridinium moieties as antimicrobial surface modifying agents.

Accordingly, the preferred embodiments provide a simplified method of preparing highly active antimicrobial agents.

In one aspect, an antimicrobial polymeric material is described, comprising a repeating unit having a formula: 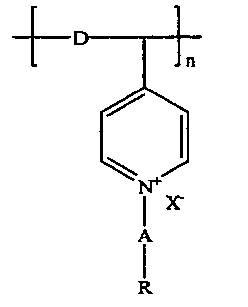 wherein R is a substituted or unsubstituted phenyl group; A is a C₁₋₆ alkyl chain; D is a C₁₋₆ alkyl chain; X is halogen, and n is at least 2. In an embodiment described, R is a phenyl group. In another embodiment described, X is chlorine. In another embodiment described, X is bromine. In another embodiment described, A is selected from the group consisting of methylene and ethylene. In another embodiment described, D is methylene. In another embodiment described, R is a phenyl group substituted with a -(E)ₘ-Si(-O-Alk)₃ group, wherein each Alk is independently a C₁₋₆ alkyl, E is a hydrocarbyl group containing m carbon atoms, and m is from 0 to 12, for example, the -(E)ₘ-Si(-O-Alk)₃ group is a para substituent on the phenyl group, wherein each Alk is methyl and wherein - (E)ₘ- is a lower alkylene chain, or E is a group having a formula -CH₂-Ph-CH₂-CH₂-, wherein Ph is a phenyl group. In another embodiment described, n is from 2 to 1000, or from 50 to 200. In another embodiment described, a surface comprising the antimicrobial polymeric material is provided, e.g., a glass surface, a wood surface, a polymer surface, a fabric, or at least one natural fiber such as cotton fiber.

In another aspect, a method for preparing an antimicrobial polymeric material is provided, the method comprising combining a hydrocarbyl halide, a chloroalkyl-functional silane and a poly(4-vinylpyridine) in a one-pot reaction mixture, whereby a silylated quaternized salt having antimicrobial properties is obtained. In an embodiment of the aspect, the hydrocarbyl halide is of the formula CₙH₂ₙ₊₁-X, wherein n is from 1 to 18 and wherein X is chlorine or bromine. In an embodiment of the aspect, the hydrocarbyl halide is selected from the group consisting of 1-chlorobutane, 1-bromoheptane, 1-bromooctane, benzyl chloride, ethylbenzyl chloride, and allyl chloride. In an embodiment of the aspect, the haloalkylsilane is of the formula (Alk-O-)₃Si(-(E)ₘ-Hal), wherein each Alk is independently C₁₋₆ alkyl, E is a hydrocarbyl group containing m carbon atoms, m is from 0 to 12, and Hal is chlorine or bromine. In an embodiment of the aspect, the haloalkylsilane is γ-chloropropyltrimethoxysilane. In an embodiment of the aspect, the poly(4-vinylpyridine) has a molecular weight of from about 10,000 MW to about 180,000 MW, or a molecular weight of about 20,000 MW. In an embodiment of the aspect, the antimicrobial polymeric material is at least 50% quaternized.

In another aspect, a method for preparing an antimicrobial polymeric material is provided, the method comprising combining a hydrocarbyl halide and a poly(4-vinylpyridine) in a one-pot reaction mixture, whereby a silylated quaternized salt having antimicrobial properties is obtained. In an embodiment of the aspect, the hydrocarbyl halide is of the formula CₙH₂ₙ₊₁-X, wherein n is from 1 to 18 and wherein X is chlorine or bromine. In an embodiment of the aspect, the hydrocarbyl halide is selected from the group consisting of 1-chlorobutane, 1-bromoheptane, 1-bromooctane, benzyl chloride, ethylbenzyl chloride, and allyl chloride. In an embodiment of the aspect, the poly(4-vinylpyridine) has a molecular weight of from about 10,000 MW to about 160,000 MW, or about 20,000 MW. In an embodiment of the aspect, the antimicrobial polymeric material is at least 50% quaternized.

In another aspect, a method of rendering a surface resistant to microbial growth is described, the method comprising the steps of applying an antimicrobial polymeric material prepared by combining a hydrocarbyl halide, a chloroalkyl-functional silane and a poly(4-vinylpyridine) in a one-pot reaction mixture; and applying the antimicrobial polymeric material to a surface, whereby the surface is rendered antimicrobial. In an embodiment of the aspect, the surface is a cotton fiber surface. In an embodiment of the aspect, the surface is a glass surface. In an embodiment of the aspect, the microbe is a Gram-negative bacterium, e.g., *Escherichia Coli, Pseudomonas aeruginosa,* or *Klebsiela pneumoniae.* In an embodiment of the aspect, the microbe is a Gram-positive bacterium, e.g., *Staphylococcus aureus.*

In another aspect, a method of rendering a surface resistant to microbial growth is described, the method comprising the steps of applying an antimicrobial polymeric material prepared by combining a hydrocarbyl halide and a poly(4-vinylpyridine) in a one-pot reaction mixture; and applying the antimicrobial polymeric material to a surface, whereby the surface is rendered antimicrobial. In an embodiment of the aspect, the surface is a cotton fiber surface. In an embodiment of the aspect, the surface is a glass surface. In an embodiment of the aspect, the microbe is a Gram-negative bacterium, e.g., *Escherichia Coli, Pseudomonas aeruginosa,* or *Klebsiela pneumoniae.* In an embodiment of the aspect, the microbe is a Gram-positive bacterium, e.g., *Staphylococcus aureus.*

### DETAILED DESCRIPTION OF THE PREFERRED EMBODIMENT

The following description and examples illustrate some exemplary embodiments of the disclosed invention in detail. Those of skill in the art will recognize that there are numerous variations and modifications of this invention that are encompassed by its scope. Accordingly, the description of a certain exemplary embodiment should not be deemed to limit the scope of the present invention.

### Quaternized Antimicrobial Salts

A simplified method for preparing quaternized salts that resist cross-linking is provided, thereby providing a surface with more antimicrobial active groups. The functionalizing agent, a silylated PVP quatemized salt (II), is prepared by reacting an alkyl halide (1), a chloroalkyl-functional silane (e.g., γ-chloropropyltrimethoxysilane) (2); and poly(4-vinylpyridine) (3) in a one-pot reaction under anhydrous conditions.

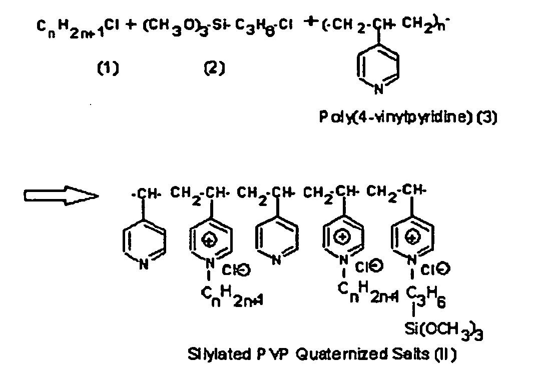

Alternatively, the reaction can be conducted in two steps by reacting the alkyl halide (1) and poly(4-vinylpyridine) (3) to yield a partially quatemized PVP salt (I). The partially quatemized PVP salt (I) can be used as an antimicrobial agent without further functionalization, or it can be reacted with the chloroalkyl-functional silane (e.g., γ-chloropropyltrimethoxysilane) (2) to yield a silylated PVP quaternized salt (II).

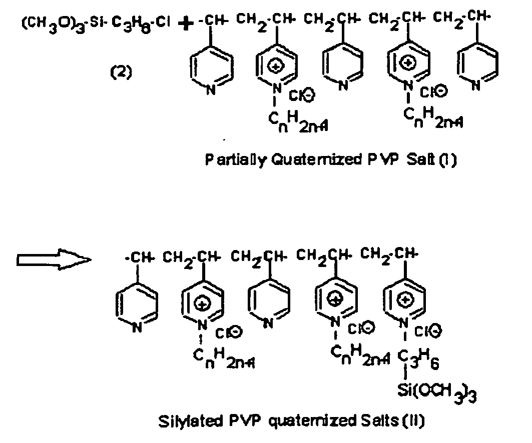

### The Hydrocarbyl Halide

Preferably, the hydrocarbyl halide is an alkyl halide of the formula CₙH₂ₙ₊₁-X, wherein X is a halogen selected from the group consisting of F, Cl, Br, and I, with Cl especially preferred. The term "alkyl" as used herein is a broad term, and is to be given its ordinary and customary meaning to a person of ordinary skill in the art (and it is not to be limited to a special or customized meaning), and refers without limitation to a straight chain or branched, acyclic or cyclic, unsaturated or saturated aliphatic hydrocarbon containing 1, 2, 3, 4, 5, 6, 7, 8, 9, 10, 11, 12, 13, 14, 15, 16, 17, 18, or more carbon atoms *(e.g.,* C₁₋₁₈ alkyl), while the term "lower alkyl" has the same meaning as alkyl but contains 1, 2, 3, 4, 5, or 6 carbon atoms *(e.g.,* C₁₋₆ alkyl), and the term "higher alkyl" has the same meaning as alkyl but contains 7 or 8 carbon atoms up to about 18 carbon atoms or higher (e.g., C₈₋₁₈ alkyl). Representative straight chain alkyls include methyl, ethyl, n-propyl, n-butyl, n-pentyl, n-hexyl, and the like; while branched alkyls include isopropyl, *sec*-butyl, isobutyl, *tert*-butyl, isopentyl, and the like. Unsaturated alkyls contain at least one double or triple bond between adjacent carbon atoms (referred to as an "alkenyl" or "alkynyl," respectively). The term "alkylene chain" as used herein is used to describe a saturated carbon chain of varying length between two other groups, e.g., methylene chain (-CH₂-), ethylene chain (-CH₂CH₂-). A "lower alkylene chain" contains, e.g., 1, 2, 3, 4, 5, or 6 carbon atoms.

The term "cycloalkyl" as used herein is a broad term, and is to be given its ordinary and customary meaning to a person of ordinary skill in the art (and it is not to be limited to a special or customized meaning), and refers without limitation to alkyls that include mono-, di-, or poly-homocyclic alkyl ring systems. Cyclic alkyl moieties (also referred to as "cycloalkyls" or "homocyclic rings) include, e.g., cyclopropyl, cyclobutyl, cyclopentyl, cyclohexyl, -CH₂-cyclopropyl, -CH₂-cyclobutyl, -CH₂-cyclopentyl, -CH₂-cyclohexyl, cyclopentenyl, and cyclohexenyl moieties. In particularly preferred embodiments, a straight chain alkyl halide is employed. Lower alkyl halides are also particularly preferred (e.g., n = 1 to 6). While alkyl halides incorporating a single halogen substituent are particularly preferred, in certain embodiments it can be acceptable or even desirable to incorporate two or more halogen substituents (either the same, or different). Particularly preferred alkyl halides include 1-chlorobutane, 1-bromoheptane, 1-bromooctane, and allyl chloride.

While alkyl halides are preferred, other hydrocarbyl halides can also be employed, e.g., aryl halides, arylalkyl halides, and alkylaryl halides. The term "aryl" as used herein is a broad term, and is to be given its ordinary and customary meaning to a person of ordinary skill in the art (and it is not to be limited to a special or customized meaning), and refers without limitation to an aromatic carbocyclic moiety such as phenyl or naphthyl, including mono-, di-, and poly-homocyclic aromatic ring systems (e.g., C₆₋₁₈ aryl). The term "arylalkyl" as used herein is a broad term, and is to be given its ordinary and customary meaning to a person of ordinary skill in the art (and it is not to be limited to a special or customized meaning), and refers without limitation to an alkyl having at least one alkyl hydrogen atom replaced with an aryl moiety, such as benzyl or naphthyl. Representative arylalkyls include -CH₂-(1-naphthyl), -CH₂-(2-naphthyl), -CH₂-(phenyl), -(CH₂)₂-(phenyl), -(CH₂)₃-(phenyl), and -CH-(phenyl)₂. The term "alkylaryl" as used herein is a broad term, and is to be given its ordinary and customary meaning to a person of ordinary skill in the art (and it is not to be limited to a special or customized meaning), and refers without limitation to an aryl having at least one aryl hydrogen atom replaced with an alkyl moiety, such as methyl. Particularly preferred arylalkyl halides include benzyl chloride, methylbenzyl chloride, and ethylbenzyl chloride.

### The Haloalkyl Silane

The chloroalkyl silane is preferably of the formula (Alk-O-)₃Si(-E-Hal) wherein Alk are the same or different alkyl groups, and Hal is halogen selected from the group consisting of F, Cl, Br, and I, with Cl especially preferred. Alk is preferably selected from lower alkyl groups, e.g., methyl. E is preferably a C₁₋₁₂ hydrocarbyl linking group, e.g., an alkyl chain, or a hydrocarbyl group having a phenyl ring linking the silane group to the rest of the polymer such as a group having a formula -CH₂-Ph-CH₂-CH₂-, wherein Ph is a phenyl group. Particularly preferred is to have methoxy groups, e.g., three methoxy groups and an n-propyl group with a terminal Cl substituent attached to the silicon atom (e.g., γ-chloropropyltrimethoxysilane).

In certain embodiments the haloalkyl silane can be omitted from the reaction mixture to obtain as the antimicrobial functionalizing agent a silane-free PVP quatemized salt.

### The Polyvinylpyridine

The poly(4-vinylpyridine) can be of any suitable chain length or molecular weight. The poly(4-vinylpyridine) has from 2 pyridinium units up to over about 1500 pyridinium units, preferably from about 100, 200, 300, 400, or 500 pyridinium units up to about 600, 700, 800, 900, or 1000 pyridinium units. Particularly preferred is a poly(4-vinylpyridine) with about 190 pyridinium units (corresponding to 20,000 MW). Such a poly(4-vinylpyridine) is commercially available as REILLINE^{™} 410 from Reilly Industries of Indianapolis, IN. In certain embodiments an even higher molecular weight poly(4-vinylpyridine) can be employed, e.g., up to about 160,000 MW or even higher.

### The Quaternized Antimicrobial Functionalizing Agent

As discussed above, in certain embodiments the haloalkyl silane can be omitted from the reaction mixture to obtain as the antimicrobial functionalizing agent a silane-free PVP quaternized salt, e.g.:

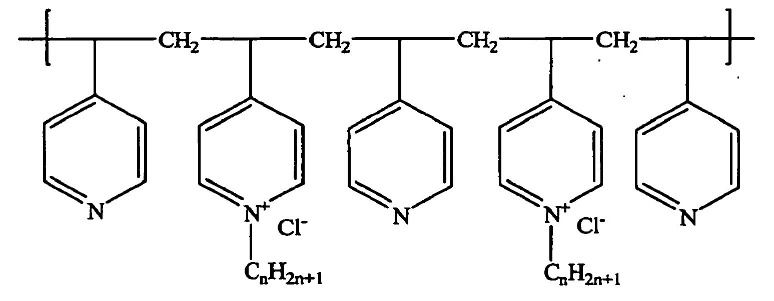

Alternatively, a haloalkyl silane can be employed in the reaction product to yield a silylated quaternized salt, e.g.:

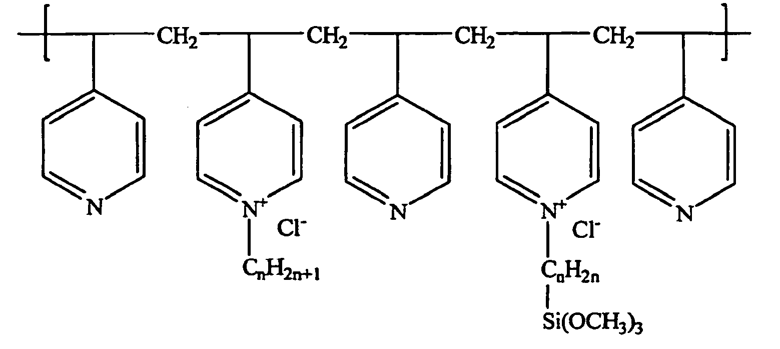

Different degrees of quaternization can be obtained by adjusting the amount of alkyl halide (or other hydrocarbyl halide) in the reaction mixture. In the two polymeric structures depicted above, a degree of quaternization of 50% is depicted. The degree of silylation in the second structure is 25%. Lower degrees of quaternization can also be advantageously employed, e.g., less than 50%, e.g., 5% to 45% or more; however, it is generally preferred to maximize quaternization, e.g., a degree of quaternization of more than 50%, e.g., up to 60%, 70%, 80%, 90%, 95%, or 100%. The degree of silylation is limited by the degree of quaternization, but advantageously silylation is also maximized, especially in those applications where excellent fasting is desirable.

A single type of repeating unit can be employed, or mixed chemistry repeating units can be obtained by using suitable mixtures of reactants with different moieties.

Advantages of the one pot reaction as described above is that it employs two fewer reaction steps than the prior art method using 3-aminopropyltrimethoxysilane and 1,4-dibromobutane. The resulting functionalized product (the silylated PVP quaternized salt) offers advantages as well, in that it is soluble in water and/or an organic solvent, such as methanol, thus rendering further handling and process steps, much easier. Such handling steps can involve coating techniques as known in the art, e.g., immersion, dipping, spraying, aerosolizing, nebulizing, brushing, curtain coating, roller painting, silk screening, lithography, ink jetting, and the like on the substrate surface. For siliceous substrates (e.g., glass) having surface silanol groups (-Si-OH), a trialkoxysilyl pendant (e.g., trimethoxysilyl pendant, -Si-(OCH₃)₃) is preferred for use in a coating material to form covalent attachment to the substrate via the silanol groups. Because such a coating is covalently bonded to the substrate, it is durable and not easily removed (e.g., by washing off).

The antimicrobial agents can be applied by any suitable method. Preferably, they are applied as a coating to a surface that comprises functional groups that can covalently bond to a corresponding functional group in the agent. Alternatively, the agents can be applied to a suitable substrate which is then transferred onto the surface to be rendered antimicrobial. The agents can be incorporated into polishes, surface cleaners, caulks, adhesives, finishes, paints, waxes polymerizable compositions (including phenolic resins, silicone polymers, chlorinated rubbers, coal tar and epoxy combinations, epoxy resin, polyamide resins, vinyl resins, elastomers, acrylate polymers, fluoropolymers, polyesters and polyurethanes, latex) for delivery to a surface to be rendered antimicrobial. A continuous or intermittent coating can be applied. The entire surface or a portion thereof can be treated.

### Applications and Materials

The antimicrobial agents can be applied by any suitable method. Preferably, they are applied as a coating to a surface that comprises functional groups that can covalently bond to a corresponding functional group in the agent. Alternatively, the agents can be applied to a suitable substrate which is then transferred onto the surface to be rendered antimicrobial. The agents can be incorporated into polishes, surface cleaners, caulks, adhesives, finishes, paints, waxes polymerizable compositions (including phenolic resins, silicone polymers, chlorinated rubbers, coal tar and epoxy combinations, epoxy resin, polyamide resins, vinyl resins, elastomers, acrylate polymers, fluoropolymers, polyesters and polyurethanes, latex) for delivery to a surface to be rendered antimicrobial. A continuous or intermittent coating can be applied. The entire surface or a portion thereof can be treated.

The antibacterial functionalizing agents of preferred embodiments can be employed in a variety of applications to prevent or inhibit microbial growth. The agents are especially effective in inhibiting the growth of Gram-positive and Gram-negative bacteria; however, growth of other microbes and microorganisms can also be prevented or inhibited, e.g., fungi such as *Candida albicans,* viruses and protists. Gram-positive bacteria have as part of their cell wall structure peptidoglycan as well as polysaccharides and/or teichoic acids and are characterized by their blue-violet color reaction in the Gram-staining procedure. Representative Gram-positive bacteria include, but are not limited to, *Actinomyces spp., Bacillus anthracis, Bifidobacterium spp., Clostridium botulinum, Clostridium perfringens, Clostridium spp, Clostridium tetani, Corynebacterium diphtheriae, Corynebacterium jeikeium, Enterococcus faecalis, Enterococcus faecium, Erysipelothrix rhusiopathiae, Eubacterium spp., Gardnerella vaginalis, Gemella morbillorum, Leuconostoc spp , Mycobacterium abcessus, Mycobacterium avium complex, Mycobacterium chelonae, Mycobacterium fortuitum, Mycobacterium haemophilium, Mycobacterium kansasii, Mycobacterium leprae, Mycobacterium marinum, Mycobacterium scrofulaceum, Mycobacterium smegmatis, Mycobacterium terrae, Mycobacterium tuberculosis, Mycobacterium ulcerans, Nocardia spp , Peptococcus niger, Peptostreptococcus spp., Proprionibacterium spp., Staphylococcus aureus, Staphylococcus auricularis, Staphylococcus capitis, Staphylococcus cohnii, Staphylococcus epidermidis, Staphylococcus haemolyticus, Staphylococcus hominis, Staphylococcus lugdanensis, Staphylococcus saccharolyticus, Staphylococcus saprophyticus, Staphylococcus schleiferi, Staphylococcus similans, Staphylococcus warneri, Staphylococcus xylosus, Streptococcus agalactiae* (group B *streptococcus), Streptococcus anginosus, Streptococcus bovis, Streptococcus cartis, Streptococcus equi, Streptococcus milleri, Streptococcus mitior, Streptococcus mutans, Streptococcus pneumoniae, Streptococcus pyogenes* (group A *streptococcus), Streptococcus salivarius,* and *Streptococcus sanguis.* Gram-negative bacteria are characterized by the presence of a double membrane surrounding each bacterial cell. Representative Gram-negative bacteria include, but are not limited to, *Acinetobacter calcoaceticus, Actinobacillus actinomycetemcomitans, Aeromonas hydrophila, Alcaligenes xylosoxidans, Bacteroides, Bacteroides fragilis, Bartonella bacilliformis, Bordetella spp., Borrelia burgdorferi, Branhamella catarrhalis, Brucella spp., Campylobacter spp., Chalmydia pneumoniae, Chlamydia psittaci, Chlamydia trachomatis, Chromobacterium violaceum, Citrobacter spp., Eikenella corrodens, Enterobacter aerogenes, Escherichia coli, Flavobacterium meningosepticum, Fusobacterium spp., Haemophilus influenzae, Haemophilus spp., Helicobacter pylori, Klebsiella spp., Legionella spp., Leptospira spp., Moraxella catarrhalis, Morganella morganii, Mycoplasma prteumoniae, Neisseria gonorrhoeae, Neisseria mertingitidis, Pasteurella multocida, Plesiomonas shigelloides, Prevotella spp., Proteus spp., Providencia rettgeri, Pseudomortas aeruginosa, Pseudomonas spp., Rickettsia prowazekii, Rickettsia rickettsii, Rochalimaea spp., Salmonella spp., Salmortella typhi, Serratia marcescens, Shigella spp., Treponema carateum, Treponema pallidum, Treponema pallidum endemicum, Treponema pertenue, Veillonella spp., Vibrio cholerae, Vibrio vulnificus, Yersinia enterocolitica,* and *Yersinia pestis.*

The agents of preferred embodiments can be applied to a wide range of surfaces to impart antibacterial effects. The agents can be employed in treating fabrics (woven or nonwoven) or fibers, e.g., collagenic (leather, suede), cellulosic (cotton, linen), and keratinic (wool, silk) materials employed in clothing, bed and bath linens, upholstery and other home furnishings (carpeting, curtains, wall coverings); synthetic fabrics and fibers (polyesters, nylon, silicones, rubbers, latex, plastics, polyanhydrides, polyorthoesters, polyamides, polyacrylonitrile, polyurethanes, polyethylene, polytetrafluoroethylene, polyphazenes, and polyethylenetetraphthalate); natural-synthetic fiber blends (e.g., polyester/cotton 65:35, DACRON®/cotton, nylon/cotton, and other cotton blends); fabrics or fibers for use in medical applications (bandages, dressings, drapes, masks, protective clothing); paper products (paper towels, tissues, sanitary products, toilet paper, stationery); glass surfaces, polymer surfaces (e.g., on or in kitchen appliances, floor and wall coverings, countertops, furniture, food storage containers, personal care product containers, utensils, medical devices, vinyl products, latex products, disposable gloves, electronics devices, computers, keyboards, cellular telephones, pagers, personal digital assistants, telephones, calculators, handheld electronics, and the like); paints and pigments; ceramic or composite surfaces (e.g., sinks, bathtubs, showers, toilets, medical devices or implants); wood (floors, furniture, trim, cabinetry, other construction materials); synthetic or natural stone (concrete, floors, countertops, tiling; construction materials, such as masonry or grout); metal (kitchen utensils, medical devices, cooking surfaces, doorknobs, countertops, industrial machinery in the sanitation, food, cosmetic, or pharmaceutical industries, medical devices, water storage tanks, piping); and the like.

The agents are particularly well suited for use in devices in the medical industry, e.g., instruments and devices, whether disposable or intended for repeated uses, scalpels, needles, scissors and other devices used in invasive surgical, therapeutic or diagnostic procedures; implantable medical devices, including artificial blood vessels, catheters and other devices for the removal or delivery of fluids to patients, artificial hearts, artificial kidneys, orthopedic pins, plates and implants; catheters and other tubes (including urological and biliary tubes, endotracheal tubes, insertable central venous catheters, dialysis catheters, long term tunneled central venous catheters, peripheral venous catheters, short term central venous catheters, arterial catheters, pulmonary catheters, Swan-Ganz catheters, urinary catheters, peritoneal catheters), urinary devices (including long term urinary devices, tissue bonding urinary devices, artificial urinary sphincters, urinary dilators), shunts (including ventricular or arterio-venous shunts); prostheses (including breast implants, penile prostheses, vascular grafting prostheses, heart valves, artificial joints, artificial larynxes, otological implants), vascular catheter ports, wound drain tubes, hydrocephalus shunts, pacemakers and implantable defibrillators, medical equipment, medical gear worn or carried by personnel in the health care setting, tubes and canisters used in respiratory treatments, including the administration of oxygen, of solubilized drugs in nebulizers and of anesthetic agents, gloves, aprons and faceshields, handles and cables for medical or dental equipment, heart valves, hip implant, dentures, crowns, braces, dental implants, drills, and other dental devices, and the like.

The agents are also useful in the food processing industry, e.g., milk, cheese, and meat processing facilities on bottling apparatus, packaging apparatus, plastic curtains, conveyor belt material, evisceration equipment, and stainless steel surfaces. Water treatment applications include water cooling towers, water heaters, water distribution conduits, municipal water storage tanks, private wells, and drip irrigation systems. The agents are also suitable for use in air filtration and treatment applications, e.g., air conditioners, air cleaner filters, ductwork, heating units, and the like.

### General Procedures

Silylated PVP quaternized salts were prepared by the following procedures. The materials used included poly(4-vinylpyridine) (42% solution in methanol, REILLINE^{™} 410, MW 20,000 from Reilly Industries, Inc., Indianapolis, IN); 3-chloropropyltrimethoxysilane (CAS 2530-87-2, KBM-703 from Shin-Etsu Chemical Co., Ltd., Tokyo, Japan); 1-chlorobutane (CAS 109-69-3 from Acros Organics, Geel, Belgium); allyl chloride (CAS 107-05-1 from Ferak Chemical Company, Berlin, Germany); 1-bromooctane (CAS 118-83-1 from Acros Organics); 1-bromoheptane (CAS 629-04-9 from Acros Organics); Benzyl chloride (CAS 100-44-7); ethylbenzyl chloride (CAS 26968-58-1), consisted of para- and ortho-isomers in about 70:30 ratio; and methanol (anhydrous).

The poly(4-vinylpyridine) was diluted with methanol to reduce the viscosity for ease of handling, then dehydrated with Molecular Sieve 4A in a sufficient amount to remove any trace of water. The solids content of the PVP was adjusted to 20%. It is noted that REILLINE^{™} 410 poly(4-vinylpyridine) used as received from the manufacturer gelled in the presence of KBM-703 3-chloropropyltrimethoxysilane. Accordingly, it is desirable to maintain the system water free or to otherwise minimize the presence of water.

A mixture of the dehydrated poly(4-vinylpyridine) (40 mmoles of vinylpyridine monomer repeating units), 30-36 mmoles of halo-alkane (C₄ - C₈), and 2-6 mmoles of KBM-703 3-chloropropyltrimethoxysilane was charged into a 50-ml Wheaton glass serum bottle, sealed with a rubber stopper and aluminum cap, then heated in an oil bath kept at 95 -100°C for 8-12 hrs. The reaction mixture changed from a two-phase liquid to a homogeneous solution as the reaction progressed and the completion of the reaction was indicated by a shift of the InfraRed (IR) peak at 1600 cm⁻¹ (pyridine ring C=N stretching band) to 1640 cm⁻¹ (after quaternization). The product thus obtained was readily soluble in water to form an amber-colored clear solution.

### Example 1

A mixture of dehydrated REILLINE^{™} 410 poly(4-vinylpyridine) (21.0 g, 40 mmoles), 1-chlorobutane (3.2 g, 33.0 mmoles), KBM-703 3-chloropropyltrimethoxysilane (1.2 g, 6.0 mmoles), and 9.0 g methanol was charged into a 50-ml serum glass bottle, sealed and reacted at 95°C for 12 hrs. The reaction mixture began as a two-phase liquid but later changed to a homogeneous mixture toward the end of the reaction. The quaternized polymers were characterized by IR spectroscopy using a KBr pellet, which showed shift of the peak from 1598 cm⁻¹ (pyridine ring C=N) to 1641cm⁻¹ (after quaternization). The product was readily soluble in water to form a light orange clear solution.

### Examples 2 to 10

The experimental procedure in each of Examples 2 to 10 was essentially the same as in Example 1, except that the 1-chlorobutane (1-ClC₄) used in Example 1 was replaced with 1-bromoheptane (1-BrC₇) in Example 2; 1-bromooctane (1-BrC₈) in Example 3; benzyl chloride (Bz Cl) in Example 4; ethylbenzyl chloride (E Bz Cl) in Example 5; allyl chloride (Ally Cl) in Example 6; methyl benzyl chloride (M Bz Cl) in Example 7; 1-chlorobutane and benzyl chloride in Example 8; 1-chlorobutane and ethylbenzyl chloride in Example 9; and 1-chlorobutane and methylbenzyl chloride in Example 10. The composition of the reaction mixtures in each of the Examples is provided in Table 1a.

**Table 1a.**

| Example | Reactants (mmoles) | | | | | | | | |
|---|---|---|---|---|---|---|---|---|---|
| | PVP | Ally Cl | 1-ClC₄ | Bz Cl | E Bz Cl | 1-BrC₈ | 1-BrC₇ | M Bz Cl | KBM-703 |
| 1 | 40 | --- | 33 | --- | --- | --- | --- | --- | 6 |
| 2 | 40 | --- | --- | --- | --- | --- | 36 | --- | 2 |
| 3 | 40 | --- | --- | --- | --- | 35.2 | --- | --- | 2 |
| 4 | 40 | --- | --- | 34 | --- | --- | --- | --- | 4 |
| 5 | 40 | --- | --- | --- | 20 | --- | --- | --- | 5 |
| 6 | 40 | 27 | --- | --- | --- | --- | --- | --- | 5 |
| 7 | 40 | --- | --- | --- | --- | --- | --- | 27 | 10 |
| 8 | 40 | --- | 13 | 13 | --- | --- | --- | --- | 10 |
| 9 | 40 | --- | 13 | --- | 13 | --- | --- | --- | 10 |
| 10 | 40 | --- | 13 | --- | --- | --- | --- | 1.3 | 10 |

### Comparative Examples

The following Comparative Examples were performed following essentially the same procedure as above, with the exception that no KBM-70.3 3-chloropropyltrimethoxysilane was employed and the REILLINE^{™} 410 poly(4-vinylpyridine) was used as received without dehydration. The composition of the reaction mixtures in each of the Comparative Examples is provided in Table 1b.

**Table 1b.**

| Example | Reactants (mmoles) | | | | | | | |
|---|---|---|---|---|---|---|---|---|
| | PVP | Ally Cl | 1-ClC₄ | Bz Cl | E Bz Cl | 1-BrC₈ | 1-BrC₇ | M Bz Cl |
| Non-silylated Analog of Ex. 1 | 40 | --- | 37.4 | --- | --- | --- | --- | --- |
| Non-silylated Analog of Ex. 2 | 40 | --- | --- | --- | --- | --- | 30 | --- |
| Non-silylated Analog of Ex. 3 | 40 | --- | --- | --- | --- | 30 | --- | --- |
| Non-silylated Analog of Ex. 4 | 40 | --- | --- | 36 | --- | --- | --- | --- |
| Non-silylated Analog of Ex. 5 | 40 | --- | --- | --- | 24 | --- | --- | --- |
| Non-silylated Analog of Ex. 6 | 40 | 30 | --- | --- | --- | --- | --- | --- |
| Non-silylated Analog of Ex. 7 | 40 | --- | --- | --- | --- | --- | --- | 37 |
| Non-silylated Analog of Ex. 8 | 40 | --- | 17.5 | 17.5 | --- | --- | --- | --- |
| Non-silylated Analog of Ex. 9 | 40 | --- | 17.5 | --- | 17.5 | --- | --- | --- |
| Non-silylated Analog of Ex. 10 | 40 | --- | 17.5 | --- | --- | --- | --- | 17.5 |

### Antimicrobial Testing

Procedures for determining the antimicrobial activity of immobilized antimicrobial agents were modeled after those of Japan Industrial Standard (JIS) Z2801 (Antimicrobial articles, antimicrobial test method and antimicrobial effect, established on December 20, 2000) and ASTM E2149-01 (Standard Test Method for Determining the Antimicrobial Activity of Immobilized Antimicrobial Agents Under Dynamic Contact Conditions).

### Preparation of the test solution

An aqueous solution was prepared by adding, in the sequence, 0.5 g of acetic acid and 2.5 g of quaternized product obtained from Example 1 in the form of a solution without isolation and purification. The solution, containing 20 wt. % active ingredient, was added to 100 ml of distilled water to provide a 0.5 % concentration of the antimicrobial solution. The solution was vigorously stirred to ensure completion of hydrolysis of the silyl functional groups, which resulted in the formation of a clear solution. The solution stayed clear for more than two weeks at ambient temperatures. Similar methodology was employed for preparing solutions of the quaternized product of Examples 2 through 10.

### Preparation of materials for antimicrobial tests

To test siliceous surfaces, glass microscope slides were used (SuperFrost^{®} Microscope Slides from Menzel GmbH, Braunschweig, Germany). The glass microscope slide was subjected to caustic washing, then was coated by dipping into the solution prepared as described above. After 30 minutes at ambient temperature the slide was removed from solution and dried in an oven at 100°C for 30 min. The process was repeated three (3) times.

Fabrics were prepared following the standard procedure of JIS L 0217 (Care Labeling of Textile Goods). The fabric employed was 100% plain (undyed), knitted natural white cotton cloth, washed with 0.1 % Tween-20 solution and autoclave sterilized before inoculated with microbes.

### Test Protocol

Following the absorption method protocol in JIS L 1902 (Testing for antibacterial activity and efficacy on textile products), the antimicrobial activity of the surfaces treated with the quaternized product of Examples 1 through 10 were quantified by percentage reduction in the number of bacterial colonies by comparing results from the test sample to simultaneously run controls. In the procedure, a 10 micro-liter aliquot of test inoculum having bacteria concentration prepared and adjusted to 1-5 x 10⁵ cells/ml (in accordance with the procedure 8.1.2 of JIS L 1902) was inoculated onto sterilized glass slide test pieces using a sterilized micropipette. The test pieces were covered tightly with sterilized thin glass sheets and incubated at 37°C for 18 h. The number of bacterial colonies was counted after washing out bacteria from each test piece with 20 ml ice-cooled physiological saline according to 10.1.3 of JIS L 1902. When the growth value F was greater than 1.5, the test was judged valid, and if F was less than 1.5, the result was discarded and retested. The bacterial strains tested included *Staphylococcus aureus* (ATCC29737) and *Escherichia coli* (ATCC10536).

The results of antimicrobial activity testing of the treated glass microscope slide surfaces against *Staphylococcus aureus* after 1, 2, and 3 washing cycles are summarized in Table 2. As demonstrated by the data, each of the quaternized products demonstrated good antimicrobial activity and durability, with the benzyl chloride derivative exhibiting particularly high antimicrobial activity and durability.

**Table 2.**

| Experiment | Reduction of *S. aureus* colonies (%) on Glass Surface | | |
|---|---|---|---|
| | Washing Cycle 1 | Washing Cycle 2 | Washing Cycle 3 |
| Example 1 (1-ClC₄) | 97.25 | 96.91 | 71.78 |
| Non-silylated Analog of Example 1 | 94.3 | 54.5 | --- |
| Example 2 (1-BrC₇) | 80.9 | 73.74 | 78.1 |
| Non-silylated Analog of Example 2 | 98.7 | 14.3 | --- |
| Example 3 (1-BrC₈) | 82.9 | 63.8 | 70.16 |
| Non-silylated Analog of Example 3 | 92.5 | 6.0 | --- |
| Example 4 (Bz Cl) | 99.45 | 97.42 | 91.29 |
| Non-silylated Analog of Example 4 | 90.75 | 58 | --- |
| Example 5 (E Bz Cl) | 98.75 | 92 | --- |
| Non-silylated Analog of Example 5 | 95.5 | 74.5 | --- |
| Example 6 (Ally Cl) | 99.3 | 97.96 | --- |
| Non-silylated Analog of Example 6 | 99.85 | 75.77 | --- |
| Example 8 (1-ClC₄ + Bz Cl) | 99.7 | 80.44 | --- |
| Non-silylated Analog of Example 8 | 99.85 | 30.07 | --- |
| Example 9 (1-ClC₄ + E Bz Cl) | 99.99 | 94.6 | --- |
| Non-silylated Analog of Example 9 | 89.19 | 28.76 | --- |

The results of antimicrobial activity testing of treated cotton fabric against *Staphylococcus aureus* after 0, 10, and 20 washing cycles is summarized in Table 3. As demonstrated by the data, each of the quaternized products demonstrated good antimicrobial activity and durability. The durability of the antimicrobial coatings, as demonstrated over repeated washing cycles, was exceptionally high.

**Table 3.**

| Experiment | Reduction of *S. aureus* colonies (%) on Cotton Fabric | | |
|---|---|---|---|
| | Washing Cycle 0 | Washing Cycle 10 | Washing Cycle 20 |
| Example 1 (1-ClC₄) | 96.53 | 99.8 | 91.33 |
| Non-silylated Analog of Example 1 | 99.92 | 99.58 | 98.2 |
| Example 4 (Bz Cl) | 97.64 | 99.5 | 98.09 |
| Non-silylated Analog of Example 4 | 93.85 | 99.3 | 97.32 |
| Non-silylated Analog of Example 5 | 99.996 | --- | 99.996 |
| Non-silylated Analog of Example 6 | 99.978 | --- | 99.997 |
| Non-silylated Analog of Example 7 | 99.971 | --- | 99.997 |
| Non-silylated Analog of Example 8 | 99.949 | --- | 99.964 |
| Non-silylated Analog of Example 10 | 99.947 | --- | 99.856 |

Antimicrobial activity of treated cotton fabric against *E. coli* after repeated washing cycles was also tested. As demonstrated by the data presented in Table 4, the tested quaternized product demonstrated excellent antimicrobial activity and durability over repeated washing cycles for cotton fabric.

**Table 4.**

| Experiment | Reduction of *E. Coli* colonies (%) on Cotton Fabric | | | | |
|---|---|---|---|---|---|
| | Washing Cycle 0 | Washing Cycle 10 | Washing Cycle 20 | Washing Cycle 30 | Washing Cycle 50 |
| Non-silylated Analog of Example 1 (1-ClC₄) | 98.94 | 99.39 | 99.11 | 98.09 | 96.93 |

The data of Table 5 demonstrates that glass surfaces treated with selected quaternized products demonstrated good antimicrobial activity against *E. coli* after a single washing cycle.

**Table 5.**

| Experiment | Reduction of *E. Coli* colonies (%) on Glass Surface Washing Cycle 1 |
|---|---|
| Example 1 (1-ClC₄) | 97.7 |
| Non-silylated Analog of Example 1 | 50 |
| Example 4 (Bz Cl) | 98.8 |
| Non-silylated Analog of Example 4 | 85.4 |
| Example 5 (E Bz Cl) | 99.6 |
| Non-silylated Analog of Example 5 | 99.6 |

Data for silane-free counterparts (non-silylated analogs) of selected quaternized products of the examples is provided in the above tables. For non-siliceous material applications (e.g., cotton fabrics or other natural fibers) they exhibit satisfactory antimicrobial activity and durability, although they were not as fasting as the silylated products. The polymeric bactericidal compositions of preferred embodiments can covalently bond to the surface of siliceous materials through silyl groups incorporated in their polymeric backbone, or attach firmly to the fabrics of natural (e.g., cotton) and synthetic fibers (e.g., T/C, a 35/65 synthetic/natural blend of poly(ethylene terephthalate) marketed under the trademark DACRON® and cotton) as well as through physical interaction that is long lasting even without silyl functional groups but with its long chain to withstand multiple washing cycles without losing antimicrobial efficacy. In contrast, short chain, the monomeric analogue benzalkonium chloride (Example 8) is not as washing resistant. The long-lasting bactericidal efficacy of the bactericidal non-silylated analogues of preferred embodiments on textiles is a most surprising feature.

For textile applications involving both natural and synthetic fibers, non-silylated PVP quaternized salts are just as washing durable as the silylated PVP quaternized salts, and thus may offer advantages in terms of cost and ease of use. The novel PVP quaternized salts of arylalkyl compounds, e.g., benzyl chloride, methyl benzyl chloride, and ethyl benzyl chloride, are effective antibacterial agents. Single forms as well as mixed forms are effective antibacterial agents. The higher degree of quaternization, the more polar the PVP quaternized salt becomes, thus favoring water solubility; however, in the textile applications, water solubility of the PVP quaternized salt is no disadvantage to washing durability once it is attached to the porous surface of the fabrics.

Tables 6 and 7 provide data regarding antimicrobial activity of selected non-silylated analogs against *E. Coli* and *K. pneumoniae* on T/C. Tables 8 and 9 provide data regarding antimicrobial activity of selected non-silylated analogs against *S. aureus* and P. *aeruginosa* on cotton fabric. Each of the non-silylated analogs tested exhibited excellent antimicrobial activity after repeated washing cycles (see data of Tables 6, 7 and 8). Table 9 provides data for microbe colony count difference between treated and untreated (control) fabrics at consecutive time intervals of 0, 6, 24, and 48 hours according to the *European Pharmacopoeia* test methodology.

**Table 6.**

| Experiment | Reduction of *E. Coli* colonies (%) on T/C | |
|---|---|---|
| | Washing Cycle 0 | Washing Cycle 20 |
| Non-silylated Analog of Example 4 | 99.979 | 99.982 |
| Non-silylated Analog of Example 8 | 99.999 | 99.74 |
| Benzalkonium Chloride | 99.999 | 80.9 |

**Table 7.**

| Experiment | Reduction of *K. pneumoniae* ATCC 4352 colonies (%) on T/C | |
|---|---|---|
| | Washing Cycle 0 | Washing Cycle 10 |
| Non-silylated Analog of Example 1 | 99.997 | 99.988 |
| Non-silylated Analog of Example 4 | 99.998 | 99.998 |

**Table 8.**

| Experiment | Reduction of *S aureus* ATCC 29737 colonies (%) on Cotton Fabric | |
|---|---|---|
| | Washing Cycle 0 | Washing Cycle 20 |
| Non-silylated Analog of Example 4 | 99.98 | 99.98 |
| Non-silylated Analog of Example 8 | 99.99 | 99.74 |
| Benzalkonium Chloride | 99.99 | 80.9 |

**Table 9.**

| Experiment | Reduction of *P. aeruginosa* ATCC 27853 colonies (%) on Cotton Fabric | | | |
|---|---|---|---|---|
| | 0 Hour | 6 Hour | 24 Hour | 48 Hour |
| Example 4 | 96.465 | 99.977 | 99.997 | 99.996 |

The functionalizing agents of the preferred embodiments exhibit antimicrobial activity for both Gram-positive (e.g. *Staphylococcus aureus)* and Gram-negative bacteria (e.g. *Escherichia Coli, Pseudomonas aeruginosa, Klebsiela pneumoniae).* They are particularly well suited for protecting siliceous-based materials, cellulosic materials, keratinic materials, and collagenic materials, e.g., textiles, leather, wood, glass, cement, stone, and other construction materials. By applying a suitable intermediate layer or subjecting the surface to intermediate functionalization, other types of surfaces (e.g., metal surfaces, polymer surfaces, and the like) can also be rendered antibacterial.

Excellent durability is exhibited by cotton fabrics treated with the functionalizing agents of preferred embodiments, both with and without silyl functional groups. The treated fabrics are able to withstand laundry-washing cycles and show utility as antimicrobial coatings for clothing such as socks and underwear.

The term "comprising" as used herein is synonymous with "including," "containing," or "characterized by," and is inclusive or open-ended and does not exclude additional, unrecited elements or method steps.

All numbers expressing quantities of ingredients, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should be construed in light of the number of significant digits and ordinary rounding approaches.

The term "comprising" as used herein is synonymous with "including," "containing," or "characterized by," and is inclusive or open-ended and does not exclude additional, unrecited elements or method steps.

All numbers expressing quantities of ingredients, reaction conditions, and so forth used in the specification are to be understood as being modified in all instances by the term "about." Accordingly, unless indicated to the contrary, the numerical parameters set forth herein are approximations that may vary depending upon the desired properties sought to be obtained. At the very least, and not as an attempt to limit the application of the doctrine of equivalents to the scope of any claims in any application claiming priority to the present application, each numerical parameter should be construed in light of the number of significant digits and ordinary rounding approaches.

The above description discloses several methods and materials of the present invention. This invention is susceptible to modifications in the methods and materials, as well as alterations in the fabrication methods and equipment. Such modifications will become apparent to those skilled in the art from a consideration of this disclosure or practice of the invention disclosed herein. Consequently, it is not intended that this invention be limited to the specific embodiments disclosed herein, but that it cover all modifications and alternatives coming within the true scope of the invention.

## Claims

1. A method for preparing an antimicrobial polymeric material, the method comprising:
combining a hydrocarbyl halide, a poly(4-vinylpyridine), and a chloroalkyl-functional silane in a one-pot reaction mixture, whereby a silylated quaternized salt having antimicrobial properties is obtained.

2. The method of Claim 1, wherein the chloroalkyl-functional silane is *y-*chloropropyltrimethoxysilane.

3. The method of Claim 1, wherein the hydrocarbyl halide is of the formula CₙH₂ₙ₊₁-X, wherein n is from 1 to 18 and wherein X is chlorine or bromine.

4. The method of Claim 1, wherein the hydrocarbyl halide is selected from the group consisting of 1-chlorobutane, 1-bromoheptane, 1-bromooctane, benzyl chloride, ethylbenzyl chloride, and allyl chloride.

5. The method of Claim 1, wherein the poly(4-vinylpyridine) has a molecular weight of from 10,000 MW to 160,000 MW.

6. The method of Claim 1, wherein the antimicrobial polymeric material is at least 50% quaternized.

7. The method of Claim 1, further comprising a step of applying the silylated quaternized salt having antimicrobial properties to a surface, whereby the surface is rendered antimicrobial.

8. A surface resistant to microbial growth, the surface comprising an antimicrobial polymeric material having a repeating unit having a formula: 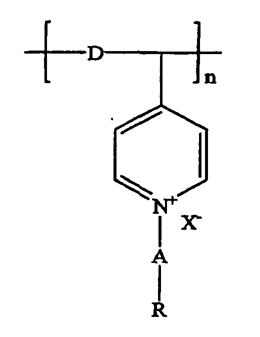 wherein R is a substituted phenyl group; A is a C₁₋₆ alkyl chain; D is a C₁₋₆ alkyl chain; X is halogen, and n is at least 2, wherein the surface is a cotton fiber surface.

## Patentansprüche

1. Verfahren zum Vorbereiten eines antimikrobiellen Polymermaterials, wobei das Verfahren enthält:
Zusammenfügen von einem Hydrocarbyl-Halid, einem Poly(4-Vinylpyridin) und einem Chloralkyl-Funktional-Silan in einer eingefäßigen Reaktionsmischung, wodurch ein silyliertes, quaternisiertes Salz erlangt wird, welches antimikrobielle Eigenschaften hat.

2. Verfahren nach Anspruch 1, bei welchem das Chloralkyl-Funktional-Silan gleich y-Chlorpropyltrimethoxysilan ist.

3. Verfahren nach Anspruch 1, bei welchem das Hydrocarbyl-Halid von der Formel CₙH₂ₙ₊₁-X ist, wobei n im Bereich von 1 bis 18 ist, und wobei X gleich Chlor oder Brom ist.

4. Verfahren nach Anspruch 1, bei welchem das Hydrocarbyl-Halid aus der Gruppe ausgewählt ist, welche 1-Chlorbutan, 1-Bromheptan, 1-Bromoktan, Benzylchlorid, Ehtylbenzylchlorid und Allylchlorid enthält.

5. Verfahren nach Anspruch 1, bei welchem das Poly(4-Vinylpyridin) ein Molekulargewicht von 10000 MW bis 160000 MW hat.

6. Verfahren nach Anspruch 1, bei welchem das antimikrobielle Polymermaterial zumindest zu 50 % quaternisiert ist.

7. Verfahren nach Anspruch 1, welches ferner einen Schritt zum Anlegen des silylierten, quaternisierten Salzes, welches antimikrobielle Eigenschaften hat, an eine Fläche enthält, wodurch die Fläche antimikrobiell erstellt wird.

8. Fläche, welche gegen mikrobielles Wachstum resistent ist, wobei die Fläche ein antimikrobielles Polymermaterial enthält, welches eine Wiederholungseinheit hat, welche die Formel hat: 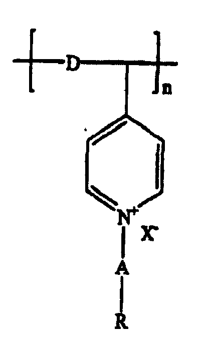 wobei R für eine Phenylgruppe steht; A eine C₁₋₆Alkylkette ist; D eine C₁₋₆Alkylkette ist; X ein Halogen ist, und n zumindest 2 beträgt, wobei die Fläche eine Baumwollfaser-Fläche ist.

## Revendications

1. Procédé de préparation d'une substance polymère antimicrobienne, le procédé comprenant :
la combinaison d'un halogénure d'hydrocarbyle, d'une poly(4-vinylpyridine) et d'un silane à fonctionnalité chloroalkyle dans un mélange réactionnel monotope, un sel quaternisé silylé ayant des propriétés antimicrobiennes étant ainsi obtenu.

2. Procédé selon la revendication 1, dans lequel le silane à fonctionnalité chloroalkyle est le γ-chloropropyltriméthoxysilane.

3. Procédé selon la revendication 1, dans lequel l'halogénure d'hydrocarbyle répond à la formule CₙH₂ₙ₊₁-X, où n vaut de 1 à 18 et où X représente le chlore ou le brome.

4. Procédé selon la revendication 1, dans lequel l'halogénure d'hydrocarbyle est choisi dans le groupe constitué par le 1-chlorobutane, le 1-bromoheptane, le 1-bromooctane, le chlorure de benzyle, le chlorure d'éthylbenzyle et le chlorure d'allyle.

5. Procédé selon la revendication 1, dans lequel la poly(4-vinylpyridine) a un poids moléculaire de 10 000 PM à 160 000 PM.

6. Procédé selon la revendication 1, dans lequel la substance polymère antimicrobienne est au moins quatemisée à 50 %.

7. Procédé selon la revendication 1, comprenant en outre une étape d'application du sel quatemisé silylé ayant des propriétés antimicrobiennes sur une surface, la surface étant ainsi rendue antimicrobienne.

8. Surface résistant à une croissance microbienne, la surface comprenant une substance polymère antimicrobienne ayant une unité répétitive répondant à la formule : 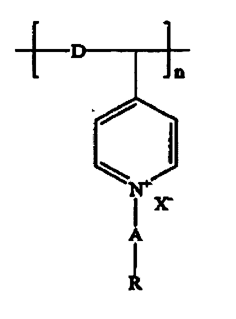 où R est un groupe phényle substitué, A est une chaîne alkyle en C₁-C₆, D est une chaîne alkyle en C₁-C₆, X est un halogène et n vaut au moins 2, la surface étant une surface de fibre de coton.
